# FASCICULE DE BREVET EUROPEEN

(11) **EP 2 574 394 B1**
(45) Date de publication et mention de la délivrance du brevet: **25.06.2014**
(21) Numéro de dépôt: 12179126.3
(22) Date de dépôt: 03.08.2012
(51) Int. Cl.: B01F 3/02, B01F 5/04

(54) **Module d'injection de gaz notamment pour installation d'administration de NO gazeux**
Gaseinspritzgerät, insbesondere für gasförmiges NO Verabreichungsgerät.
Gas injection module, in particular for NO gas administration equipment.

(30) Priorité: 29.09.2011 FR 1158724
(43) Date de publication de la demande: 03.04.2013
(73) Titulaire: Air Liquide Medical Systems, 92160 Antony (FR)
(72) Inventeur: Boulanger, Thierry, 37000 TOURS (FR); Giard, Pauline, 14190 SAINT GERMAIN LE VASSON (FR); Libardi, Mickaël, 94270 LE KREMLIN-BICETRE (FR)
(74) Mandataire: Pittis, Olivier

(56) Documents cités:
- EP-A1- 2 255 867
- EP-A2- 1 726 355
- WO-A1-2007/115810
- FR-A- 1 523 920
- JP-A- 59 162 932

## Description

L'invention concerne un module d'injection de gaz pour dispositif de délivrance de mélange gazeux à un patient à des fins d'assistance, de monitorage ou de diagnostic de ses fonctions respiratoires, ledit module d'injection de gaz étant destiné à être raccordé, d'une part, à une source de gaz thérapeutique, tel un appareil de délivrance de mélange NO/N₂, et, d'autre part, au circuit ventilatoire d'un ventilateur d'administration de gaz, tel un ventilateur délivrant un mélange air/O₂ ou N₂/O₂, de manière à pouvoir réaliser un mélange homogène de ces gaz, en particulier pour réaliser ensuite une administration par voie inhalée de monoxyde d'azote NO à un patient.

L'oxyde nitrique ou monoxyde d'azote, appelé ci-après « NO », est un gaz incolore qui, lorsqu'il est inhalé, dilate les vaisseaux sanguins des poumons et augmente l'oxygénation du sang en favorisant les échanges gazeux. Cette propriété est exploitée pour traiter l'hypertension artérielle pulmonaire.

A cette fin, le NO, typiquement en mélange avec de l'azote (N₂), est ajouté aux gaz inspirés par le patient souffrant d'hypertension pulmonaire selon une posologie déterminée, fixée par le médecin, qui varie généralement entre 1 et 100 ppm en volume, notamment en fonction du patient considéré, typiquement entre 10 et 50 ppm vol.

Le NO est délivré par un dispositif de délivrance, comme décrit par exemple par US-A-5,558,083. Un tel appareil, qui est alimenté par une ou des bouteilles contenant un mélange d'azote (N₂) et de NO à une concentration typiquement de l'ordre de 200 à 1000 ppm en volume de NO, comprend généralement un module d'injection de NO directement placé dans la branche inspiratoire d'un circuit patient connecté à ses extrémités respectivement à un ventilateur mécanique et à un patient.

Le module d'injection inclut un capteur de débit qui mesure le débit délivré par le ventilateur mécanique et retourne cette mesure à l'appareil d'administration de NO pour que celui-ci en déduise un débit de NO à délivrer, en relation avec la posologie désirée.

Le débit ainsi déterminé est assuré au moyen d'une électrovanne proportionnelle associée à un capteur de débit interne à l'appareil et est administré au moyen d'une ligne d'injection située dans ledit module d'injection, en amont ou en aval du système de mesure de débit.

Le mode d'administration du NO est préférentiellement continu en ce sens que l'appareil recueille de manière continue la mesure de débit du gaz circulant dans la branche inspiratoire du circuit patient et actualise en permanence le débit de NO à délivrer afin de respecter la posologie désirée.

Or, il apparaît que l'admission de NO est usuellement réalisée en un unique point, ce qui pose alors des problèmes d'homogénéité du mélange gazeux. Ceux-ci sont détaillés au travers du document US-A-5,722,392, qui met en évidence des variations de concentration en NO d'un rapport 100 entre les différents points d'un plan de coupe en aval du point d'injection.

De fait, il est alors usuel d'observer une distance minimale de plusieurs dizaines de centimètres entre le module d'injection et le patient afin que l'écoulement des gaz, turbulent, participe à fournir au patient un fluide correctement mélangé.

Pour répondre d'autre part à des exigences sécuritaires, un appareil de délivrance de NO dispose généralement d'une ligne ou d'un module de prélèvement de gaz permettant de prélever une portion du gaz inspiré par le patient, typiquement 100 ml/min environ, et préférentiellement raccordée au plus près du patient, afin de l'analyser et de déterminer les teneurs en gaz dans le circuit patient.

En d'autres termes, les appareils de délivrance de NO comprennent non seulement un module d'injection situé bien en amont du patient mais aussi un module de prélèvement des gaz inspiré situé, lui, au plus près du patient.

Considérant que chacun de ces modules est constitué d'un ou de plusieurs raccords pneumatiques et électriques connectés au dispositif d'admission du NO, il apparaît qu'une telle configuration complexifie le champ opératoire de la thérapie.

De plus, il convient de prendre également en compte les propriétés du NO qui, sous l'effet de l'oxygène, s'oxyde pour former un composé hautement toxique, à savoir le dioxyde d'azote NO₂. Il convient donc d'utiliser un analyseur de gaz raccordé au module de prélèvement pour mesurer en permanence les concentrations en NO₂ dans le gaz inhalé afin de s'assurer qu'elles sont sous un seuil critique et donc d'éviter au patient un effet délétère d'un mélange non maîtrisé trop riche en NO₂.

La cinétique d'une telle réaction dépend notamment de la posologie en NO ainsi que de la concentration en oxygène du gaz inhalé, lequel peut être enrichi par le ventilateur mécanique.

A titre informatif, sous une posologie de 10 ppmv et une concentration en oxygène de 50% en volume, 30 secondes sont suffisantes pour observer la formation de NO₂ à hauteur de 0,2 ppmv, alors que les premiers signes de bronchoconstriction apparaissent à partir de 0,6 ppmv.

Cette durée peut dès lors être considérée comme le temps maximal de transport du gaz entre le module d'injection et le patient.

Or, ce temps est fonction du débit délivré par le ventilateur mécanique, lequel peut être très faible en particulier dans le cadre d'une ventilation de nouveau-nés.

Il s'ensuit donc que plus le module d'injection de NO est loin du patient plus les risques de voir se former des radicaux en la présence du NO₂ sont élevés.

Aussi, le document US-A-5,722,392 enseigne un dispositif d'injection situé à proximité du patient, dans le but de limiter la formation de NO₂. Dès lors, il s'agit d'homogénéiser le mélange sur une courte distance entre l'injecteur et le patient.

Cependant, la solution proposée par ce document n'est pas idéale car elle génère une résistance à l'écoulement inacceptable, réduisant de fait l'efficacité du respirateur ou augmentant l'effort du patient. De plus, la solution exposée nécessite tout de même un dispositif de longueur relativement importante pour que les turbulences engendrées suffisent à homogénéiser le mélange, et est donc peu satisfaisante en termes d'intégration et de réduction du temps d'exposition du NO à l'oxygène.

Par ailleurs, le document CA-A-2691377 propose un dispositif à injection antiparallèle - par rapport au flux de gaz issu du respirateur - réduisant ainsi la taille du dispositif, et avec une faible résistance à l'écoulement. Toutefois, les posologies couramment employées lors des thérapies à base de NO inhalé varient entre 1 et 100 ppm en volume de NO, et pour des bouteilles de concentration entre 100 et 1000 ppmv, nécessitant donc une dilution d'un rapport 1 à 1000 du NO de la bouteille.

Considérant par ailleurs que les débits inspiratoires usuels varient également dans une très grande plage, typiquement de 0.5 à 200 l/min, selon le patient, le débit de gaz secondaire à administrer est nécessairement très variable d'un patient à un autre, et peut descendre jusqu'à 0.01 l/min. Le dimensionnement de la ligne d'injection devient alors délicat et il semble difficile d'obtenir un mélange homogène dans toutes les configurations, en particulier pour les très faibles débits. Le renfort d'un déflecteur semble d'une efficacité modérée dans ces mêmes cas. D'autres modules d'injection sont connus des documents FR-A-1523920, EP-A-2255 867 et EP-A-1726355.

Ainsi, il apparaît immédiatement que les solutions précédemment citées ne permettent pas de satisfaire à la fois aux contraintes de faible résistance à l'écoulement, de compacité du dispositif et de performances pour l'ensemble des posologies considérées.

Partant de là, le problème à résoudre est de proposer un dispositif de réalisation d'un mélange de plusieurs gaz à un patient, en particulier un mélange inhalable à base de NO gazeux, lequel dispositif soit de conception simple et d'encombrement limité, présentant une faible résistance à l'écoulement du gaz inspiratoire, et permette de réaliser aisément et efficacement un mélange homogène de différents composés gazeux et de s'assurer par ailleurs que le mélange gazeux ainsi réalisé correspond bien au mélange souhaité et/ou ne contient pas une ou des composés indésirables ou toxiques en concentration excessive, en particulier du NO₂ lorsque le gaz considéré est un mélange à base notamment d'oxygène et de NO.

La solution de l'invention est un module d'injection de gaz comprenant :
- un corps de module traversé par une ligne d'acheminement de gaz entre une extrémité d'entrée de gaz et une extrémité de sortie de gaz, ladite ligne d'acheminement de gaz étant délimitée par une paroi périphérique, et
- un injecteur de gaz formé d'un élément creux comprenant un orifice d'entrée de gaz permettant à un gaz de pénétrer dans l'élément creux et plusieurs orifices de sortie de gaz permettant au gaz de sortir de l'élément creux,
   et dans lequel :
- le corps de module comporte un premier orifice latéral aménagé dans la paroi périphérique de la ligne d'acheminement de gaz et au moins un deuxième orifice latéral aménagé dans la paroi périphérique de la ligne d'acheminement de gaz,
- ledit injecteur de gaz est agencé dans la ligne d'acheminement de gaz de sorte que le premier orifice latéral soit situé en vis-à-vis de l'orifice d'entrée de gaz de l'élément creux et qu'au moins une partie des orifices de sortie de gaz soient agencés de manière à distribuer du gaz en plusieurs points d'injection de la section de la ligne d'acheminement de gaz, et
- ledit au moins un deuxième orifice latéral étant aménagé dans la paroi périphérique de la ligne d'acheminement de gaz, entre l'injecteur de gaz et l'extrémité de sortie de gaz de ladite ligne d'acheminement de gaz.

Selon le cas, le module de l'invention peut comprendre l'une ou plusieurs des caractéristiques techniques suivantes :
- l'injecteur de gaz comporte entre 5 et 100 orifices de sortie de gaz.
- ledit injecteur de gaz a une forme annulaire ou semi-annulaire.
- les orifices ont un diamètre compris entre 0.1 et 3 mm.
- l'injecteur de gaz a une forme de roue comprenant un cadre creux périphérique et *n* rayons creux, avec *n* compris entre 3 et 20.
- l'injecteur de gaz a une forme de roue comprenant un cadre creux périphérique et des rayons creux, lesdits orifices de sortie de gaz étant agencés dans le cadre creux périphérique et dans les rayons creux.
- la somme des surfaces évidées situées dans le cadre creux représente au moins 50% de la section de passage de la ligne d'acheminement, de préférence au moins 70%.
- le corps de module comporte un deuxième orifice latéral et au moins un troisième orifice latéral aménagés dans la paroi périphérique de la ligne d'acheminement de gaz, au moins un desdits orifices latéraux étant agencé entre l'injecteur de gaz et l'extrémité d'entrée de gaz de la ligne d'acheminement de gaz, et au moins un desdits orifices latéraux étant agencé entre l'injecteur de gaz et l'extrémité de sortie de gaz de la ligne d'acheminement de gaz.
- le corps de module comporte au moins un deuxième orifice latéral, un troisième orifice latéral et un quatrième orifice latéral aménagés dans la paroi périphérique de la ligne d'acheminement de gaz, le deuxième orifice latéral et le troisième orifice latéral étant agencés entre l'injecteur de gaz et l'extrémité de sortie de gaz de la ligne d'acheminement de gaz, et le quatrième orifice latéral étant agencé entre l'injecteur de gaz et l'extrémité d'entrée de gaz de la ligne d'acheminement de gaz.
- un dispositif de prélèvement de gaz est agencé dans la ligne d'acheminement de gaz entre l'injecteur de gaz et l'extrémité de sortie de gaz de la ligne d'acheminement de gaz, ledit dispositif de prélèvement de gaz coopérant avec le deuxième orifice latéral.
- le dispositif de prélèvement de gaz est formé d'un second élément creux comprenant plusieurs orifices d'entrée de gaz permettant à du gaz circulant dans la ligne d'acheminement de gaz de pénétrer dans l'élément creux et un orifice de sortie de gaz permettant au gaz de sortir de l'élément creux via le deuxième orifice latéral.
- l'injecteur de gaz et le dispositif de prélèvement de gaz sont identiques.

L'invention concerne aussi une installation de distribution d'un mélange gazeux comprenant un premier gaz et un deuxième gaz, comportant :
- une première source de gaz pour distribuer un premier gaz dans une ligne principale de distribution de gaz reliée fluidiquement à ladite première source de gaz,
- un module d'injection de gaz selon l'invention agencé sur la ligne principale de distribution de gaz de manière à ce que la ligne principale de distribution de gaz et la ligne d'acheminement de gaz du module soient en communication fluidique, et
- une deuxième source de gaz en communication fluidique, via le premier orifice latéral, avec l'orifice d'entrée de gaz de l'injecteur de gaz de manière à alimenter l'injecteur de gaz en un deuxième gaz issu de la deuxième source de gaz.

Selon le cas, l'installation de l'invention peut comprendre l'une ou plusieurs des caractéristiques techniques suivantes :
- la première source de gaz comprend un ventilateur respiratoire délivrant de l'air enrichi ou non en oxygène, ou un mélange N₂/O₂.
- la deuxième source de gaz comporte est une source de NO ou d'un mélange gazeux contenant du NO.
- elle comporte en outre un humidificateur de gaz agencé entre la première source de gaz et le module d'injection, de préférence elle comporte en outre un raccord à 3-voies, telle une pièce en Y ou en T, agencé à l'extrémité aval de la canalisation principale, le module d'injection étant agencé entre l'humidificateur et le raccord à 3-voies.
- elle comporte en outre une ligne de prélèvement de gaz connectée au deuxième orifice latéral, et/ou des lignes de mesure de pression connectées au troisième et au quatrième orifice latéral.

De façon générale, le module d'injection selon l'invention est situé au plus près du patient et remplit de manière simultanée le rôle d'injection et d'homogénéisation du mélange gazeux, puis le prélèvement à des fins d'analyse permettant ainsi de limiter et contrôler la formation de NO₂ en diminuant le temps de transport des gaz au patient, et par ailleurs, de simplifier le champ opératoire en ne proposant qu'un seul et unique module.

L'invention consiste en un dispositif d'injection proximal de gaz, en particulier de monoxyde d'azote (NO), à des fins thérapeutiques permettant l'adjonction homogène d'une quantité de gaz thérapeutique, tel du NO, dans un flux de gaz principal, par exemple le flux inspiratoire issu d'un respirateur, en ventilation invasive ou non-invasive, avec un circuit patient double branche à valve, simple branche à valve ou simple branche à fuite, ou par exemple dans l'air inspiré spontanément par le patient au travers de l'invention.

L'homogénéisation du mélange gazeux est obtenue, en conservant une faible résistance à l'écoulement du flux principal et dans un faible volume, par une injection multipoints sur toute la section de la ligne du flux principal.

Il est à noter que dans le cadre de la présente invention, par « NO », on entend du NO pur ou un mélange gazeux à base de NO, c'est-à-dire contenant du NO en tant que principe actif, ce qui n'exclut pas la présence d'un ou plusieurs autres composés dans le mélange considéré, notamment de l'azote. Ainsi, une « source de NO » signifie une « source d'un mélange gazeux à base de NO », par exemple un mélange NO/N₂.

L'invention va maintenant être expliquée plus en détail en références aux Figures annexées parmi lesquelles :
- La figure 1a est une représentation schématique d'une installation d'administration de NO à un patient sous assistance respiratoire, telle que connue dans l'état de la technique.
- La figure 1b est une représentation schématique d'une administration proximale de NO à un patient sous assistance respiratoire, selon la présente invention.
- La figure 1c est une représentation schématique d'une administration proximale de NO à un patient sans assistance respiratoire, c'est-à-dire en ventilation naturelle, selon la présente invention.
- La figure 2 est une représentation schématique des entrées et sortie de gaz selon la présente invention.
- La figure 3a est une vue en perspective d'un premier mode de réalisation d'un injecteur sous la forme d'une roue d'injection à 5 rayons.
- La figure 3b est une vue en coupe de l'injecteur illustré en figure 3a.
- La figure 4 est une représentation schématique du dispositif selon la présente invention selon un deuxième mode de réalisation qui associe un injecteur pour homogénéiser le mélange à une pièce de prélèvement pour un prélèvement représentatif du gaz mélangé.
- La figure 5 est une représentation schématique selon un mode de réalisation du dispositif de prélèvement selon la présente invention.
- La figure 6 est une représentation schématique du dispositif selon la présente invention selon un deuxième mode de réalisation avec injecteur et mesure de débit par pression différentielle.
- La figure 7 est une représentation schématique du dispositif selon la présente invention selon un troisième mode de réalisation avec injecteur et une mesure de débit par un organe de mesure de débit situé dans un conduit de dérivation.
- La figure 8a est une représentation obtenue par simulation numérique d'une injection de NO telle que connue dans l'état de la technique.
- Et la figure 8b est une représentation obtenue par simulation numérique d'une injection de NO selon la présente invention.

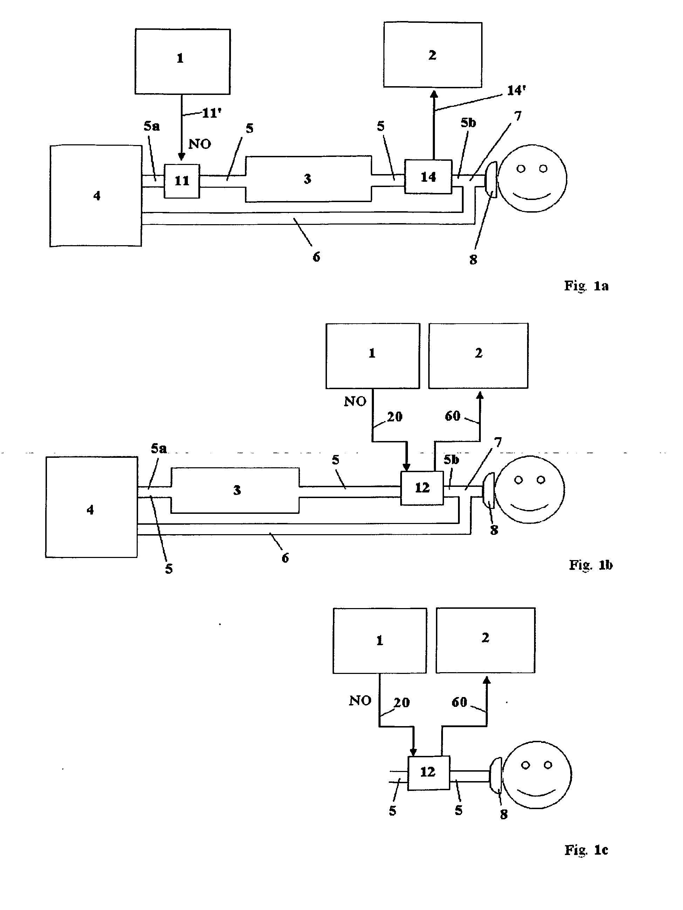

Comme illustré en Figure 1a, les installations d'administration de gaz utilisées dans les thérapies par NO inhalé utilisent habituellement une source 1 de NO, tel un module d'injection de NO, dans l'une 5 des branches du circuit patient 5, 6 dont le point d'injection 11 est situé en aval du ventilateur 4 mais en amont de l'humidificateur 3 de gaz, et un module ou dispositif de prélèvement 2 des gaz administrés au patient permettant de prélever du gaz dans le conduit 5 et de procéder à son analyse pour en déterminer des concentrations en NO, NO₂ et O₂. Le point de prélèvement 14 du dispositif de prélèvement 2 des gaz administrés est généralement situé en aval de l'humidificateur 3 et en amont de l'interface 8, tel un masque, une canule ou analogue, permettant de distribuer le gaz chargé en NO au patient, par exemple un mélange air/NO.

Le module d'injection 1 de NO comprend classiquement un capteur de débit (non représenté) qui mesure (en 11) le débit issu du ventilateur 4 pour en déduire la quantité de NO à injecter. La technologie utilisée dans les appareils connus est de type anémométrique, c'est-à-dire basée sur l'utilisation d'un fil chaud pour effectuer la mesure. Or, un tel fil chaud est particulièrement sensible à l'humidité car les gouttelettes d'eau contenues dans le gaz réchauffé par l'humidificateur 3 peuvent le détériorer, rendant alors la mesure impossible et par conséquent, provoquer l'arrêt de la thérapie.

De là, afin de protéger ce capteur, le module d'injection 1 est systématiquement placé en sortie du ventilateur 4 (raccordé en 11) et donc en amont de l'humidificateur 3.

En effet, ceci permet non seulement d'éviter une dégradation du fil chaud par de l'eau mais aussi de garantir que le NO injecté se répartisse de façon homogène dans le flux gazeux, tel de l'air ou un mélange N₂/O₂, issu du ventilateur 4 et véhiculé par la branche ou conduit 5.

Par ailleurs, le module de prélèvement 2 est quant à lui relié à ou placé dans la branche inspiratoire 5, au plus près du patient, c'est à dire juste en amont (en 14) de la pièce 7 en Y servant à relier la branche inspiratoire 5 et la branche expiratoire du circuit patient 5,6 à l'interface 8 alimentant le patient en gaz, comme illustré en Figure 1a.

Ce type de configuration présente toutefois un certain nombre d'inconvénients.

Tout d'abord, le fait de devoir utiliser plusieurs modules 1, 2, à savoir un module d'injection 1 de NO et un module de prélèvement 2, conduit à un encombrement accru du dispositif du fait de la duplication des modules 11 , 14, des lignes d'injection 11' et prélèvement 14'.

Ensuite, l'augmentation du temps de transfert du NO injecté sur toute la longueur de la branche inspiratoire 5 et son passage dans l'humidificateur 3, engendre une oxydation accrue du NO avec formation possible et indésirable de NO₂ qui est toxique à faible dose, c'est-à-dire dès à peine quelques ppm en volume.

Enfin, avec les modules connus, une homogénéité du mélange n'est pas forcément assurée car le gaz issu du respirateur n'est pas nécessairement turbulent.

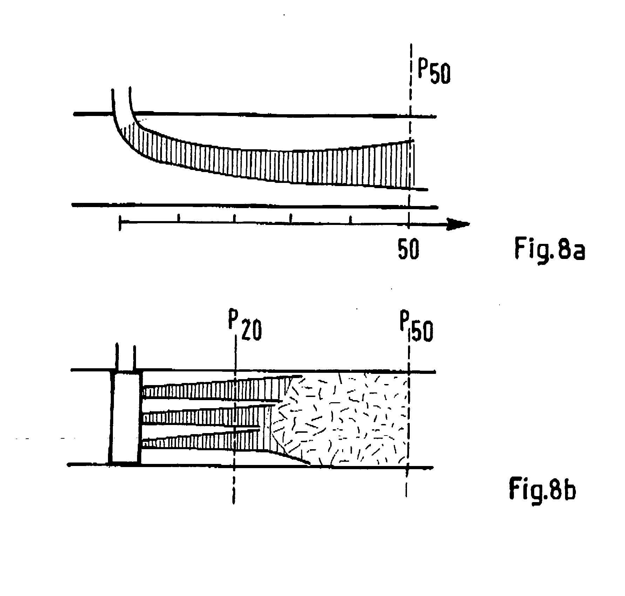

La figure 8a représente le résultat d'une simulation numérique de l'injection d'un débit de NO dans un débit d'air non turbulent à pression atmosphérique et 25°C, au travers d'un orifice 23 situé dans la paroi 15 du module d'injection 11, comme obtenu dans les appareils connus de distribution de NO. Comme on le voit, le NO délivré s'écoule dans le flux d'air sans se mélanger avec ce dernier, si bien que le mélange de NO et d'air est non homogène dans un plan P50 situé à 50 mm en aval du plan d'injection.

Comme déjà dit, la présente invention vise à résoudre tout ou partie de ces problèmes.

Ainsi, la Figure 1b est une représentation schématique d'une administration proximale de NO à un patient sous assistance respiratoire avec module d'injection 12 selon la présente invention, alors que la Figure 1c est une représentation schématique d'une administration proximale de NO à un patient respirant naturellement avec, là aussi, module d'injection 12 selon la présente invention.

Les modules connus selon ce principe ne garantissent pas un mélange homogène pour toutes les posologies car les débits en jeu sont parfois très faibles.

Afin de résoudre notamment les problèmes susmentionnés tout en garantissant une injection efficace du NO, selon l'invention, on propose un dispositif ou module d'injection 12 amélioré, venant s'insérer sur la ligne principale 5 de gaz, en aval de l'humidificateur 3 permettant de réaliser :
- une injection d'un gaz thérapeutique, dit « gaz secondaire » ou « second gaz », tel du NO ou un mélange NO/N₂, dans la ligne principale 5 du circuit patient 5, 6 qui véhicule un gaz ou mélange gazeux principal, dit « gaz principal » ou « premier gaz », tel de l'air, de l'air enrichi en O₂, ou encore un mélange N₂/O₂,
- un prélèvement efficace d'une partie du mélange gazeux formé desdits gaz principal et secondaire dans la ligne principale 5 du circuit patient de manière à s'assurer que le mélange gazeux réalisé par l'injecteur 40 est à la concentration souhaitée et ne contient pas une teneur toxique de NO₂.
- un prélèvement d'une fraction homogène du gaz là où les dispositifs de l'art antérieur mesurent potentiellement une portion non homogène et donc non représentative du mélange gazeux.

En d'autres termes, la présente invention propose, de manière générale, de fusionner les deux modules d'injection et de prélèvement classiquement 11, 14 de la Figure 1a, pour obtenir un module unique 12 situé au plus près du patient sur la canalisation principale d'acheminement 5 du gaz au patient, c'est-à-dire se présentant de préférence sous une forme d'un boitier raccordable venant se positionner sur et se raccorder fluidiquement à la canalisation principale 5 d'acheminement du gaz au patient, et aussi de concevoir le module d'injection 12 de telle sorte qu'un mélange homogène des gaz à mélanger, notamment du NO et de l'air, puisse y être réalisé et ensuite prélevé sous forme d'une portion homogène et donc représentative dudit mélange gazeux.

Plus précisément, que ce soit dans le mode de réalisation de la Figure 1b ou de celui de la Figure 1c, la ligne principale 5 dans laquelle circule le premier gaz ou gaz principal, en particulier un mélange homogène d'air et d'oxygène, lequel s'écoule depuis le ventilateur 4 vers le patient, reçoit le gaz secondaire, en particulier un mélange homogène NO/N₂, via l'injecteur 40 qui est raccordé à une entrée de gaz secondaire 23 réalisée dans la paroi 28 du module 12 reliée fluidiquement, via une ligne 20 d'alimentation en gaz secondaire, à une source de gaz secondaire, tel un dispositif d'administration de NO, à dose pulsée ou en continu, lui-même raccordé à une ou plusieurs bouteilles de mélange NO/azote, par exemple des mélanges NO/azote contenant entre 100 et 1000 ppm vol de NO, le reste étant de l'azote.

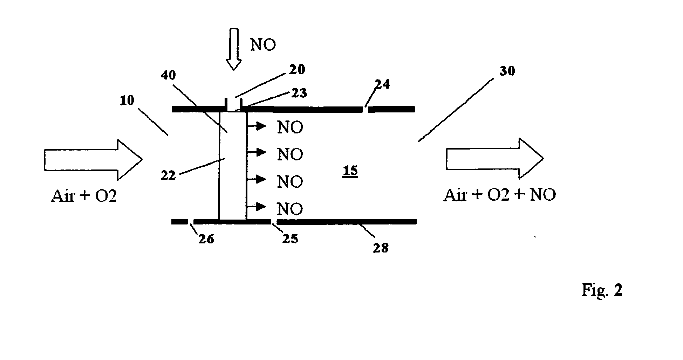

Selon l'invention, comme illustré sur les Figures 1b, 1c et 2, la ligne de gaz secondaire 20 amenant le NO est donc reliée fluidiquement à un injecteur 40 du module d'injection 12 unique, placé sur la ligne principale 5.

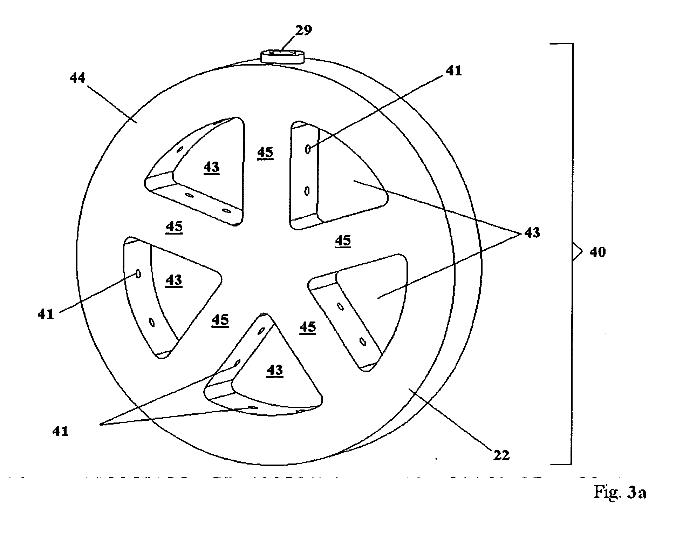

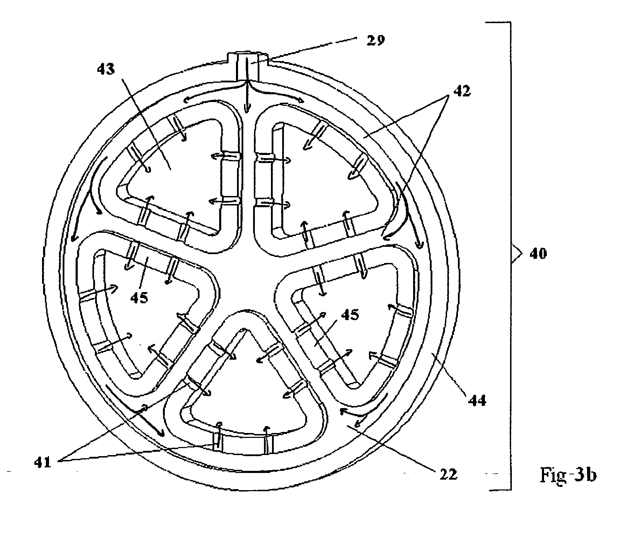

La ligne de gaz secondaire 20 permet donc d'apporter le gaz secondaire, tel un mélange NO/N₂, et de l'introduire dans l'injecteur 40 via l'orifice 23 pratiqué dans la paroi du conduit 15 du module 12 et situé en regard de l'orifice d'entrée 29 de l'injecteur 40, comme illustré en Figure 3a et 3b.

En outre, l'injecteur 40 est muni de plusieurs orifices d'injection 41 permettant la sortie du gaz secondaire et sa distribution dans le flux de gaz principal en plusieurs points de la section de la ligne ou conduite d'acheminement de gaz 15 du module 12, comme montré en Figures 3a et 3b.

Le fait d'utiliser plusieurs orifices d'injection 41, de préférence uniformément répartis sur l'injecteur 40, permet de réaliser une distribution régulière du deuxième gaz, tel un mélange NO/N₂, en plusieurs sites au sein de l'écoulement gazeux formé par le premier gaz, tel de l'air enrichi en O₂, et d'améliorer considérablement l'homogénéité de mélange des deux gaz.

Au final, le mélange gazeux homogène obtenu est administré par inhalation au patient via une interface patient 8, tel un masque ou une canule respiratoire, comme montré en Figures 1b et 1c.

Par ailleurs, comme illustré en Figures 3a et 3b, l'injecteur 40 se présente sous la forme d'un élément creux, tel un anneau ou une structure en forme de roue comme détaillé ci-après, apte à véhiculer le gaz secondaire depuis l'orifice d'entrée 29 de l'injecteur 40 jusqu'aux orifices d'injection 41 au travers de conduits 42 internes situés dans le corps 22 de l'injecteur 40.

Afin de garantir une bonne homogénéisation du mélange des gaz primaire et secondaire, les orifices 41 sont répartis sur l'ensemble de la section et sont en nombre élevé, typiquement entre 5 et 100, par exemple de 30 à 80 environ. Bien entendu, il est également possible d'utiliser un injecteur 40 comprenant plus de 100 orifices 41.

De façon générale, le gaz principal est typiquement à une pression comprise entre 0 et 100 cm H₂0, à un débit de 0.5 à 200 l/min et à une température de 10 à 40°C.

Afin de limiter la résistance à l'écoulement du gaz principal induit par la présence de l'injecteur 40 dans la ligne ou conduit d'acheminement 15, l'injecteur 40 comprend de nombreuses zones évidées 43, au travers desquelles le gaz principal circule, de manière à limiter les pertes de charge.

Ainsi, si l'on considère une section de la ligne 15, typiquement comprise entre 0.5 et 20 cm², plus particulièrement 3.5 cm², la surface évidée 43 représente une fraction importante de la surface totale, c'est-à-dire au moins 1,8 cm², typiquement au moins 2,5 cm².

De là, de manière générale, la somme de toutes les surfaces évidées 43 représente une fraction supérieure à 50%, voire supérieure à 70%, idéalement supérieure à 80% de la surface totale de la section de passage de la ligne d'acheminement 15.

Dans un mode de réalisation préféré, illustré en Figures 3a et 3b, l'injecteur 40 a une structure générale tridimensionnelle en forme de roue comprenant un cadre périphérique annulaire 44 portant *n* rayons 45 creux (n étant compris entre 3 et 20), par exemple 5 rayons. Ceci permet de disposer les orifices d'injection 41 sur toute la surface de la section de la ligne principale 5, tout en conservant une part importante de zones évidées 43, c'est-à-dire à la fois sur le cadre 44 formant pourtour annulaire et sur les rayons 45.

Afin d'augmenter le nombre d'orifices 41 sans diminuer la surface évidée totale 43 et ainsi d'obtenir une meilleure homogénéisation, on peut disposer les orifices d'injection 41 dans différents plans-sections de la ligne principale 5. Les orifices 41 peuvent être disposés selon une direction quelconque par rapport à la direction du flux principal.

Dans tous les cas, les orifices d'injection 41 permettent l'injection du gaz secondaire dans le gaz principal. Une solution préférée consiste toutefois à disposer les orifices d'injection 41 de telle manière que le gaz secondaire, tel du NO, soit délivré dans un plan perpendiculaire au flux principal, c'est-à-dire à perpendiculaire l'axe du conduit 15.

Il est à noter par ailleurs que les orifices 41 ont typiquement un diamètre compris entre 0.1 et 3 mm, idéalement inférieur à 1 mm. Les orifices 41 sont tous de mêmes diamètres ou peuvent avoir des diamètres différents, en particulier des diamètres variant avec leur disposition sur l'injecteur 40.

A titre d'exemple, sur la figure 3a a été représenté un injecteur 40 en forme de roue creuse comprenant un cadre circulaire 44 creux périphérique portant 5 branches ou rayons 45 creux, au travers desquels sont percés une trentaine d'orifices d'injection 41, disposés dans un plan unique et équi-répartis sur chacun des rayons 45 de la roue et sur son cadre 44. Une telle configuration permet un mélange homogène des gaz dans le passage 15.

La figure 3b montre l'injecteur 40 de la figure 3a vu en coupe. Comme déjà mentionné, l'injecteur 40 est creux en son sein pour permettre de transporter le gaz secondaire de l'orifice d'entrée 29 aux différents orifices d'injection 41 par l'intermédiaire de conduits internes 42.

Afin de conserver une grande surface totale évidée 43, les conduits internes 42 par lesquels le gaz secondaire est transporté jusqu'aux orifices 41 sont de faible section de passage, typiquement de section de passage inférieure à 3 mm, idéalement inférieure à 1 mm.

Or, une faible section oppose une résistance à l'écoulement du gaz secondaire dans l'injecteur 40 de sorte que les orifices 41 situés à proximité de l'entrée 29 de gaz secondaire sont parcourus par un débit de gaz supérieur à celui des orifices les plus éloignés. Dans certaines géométries, l'injecteur 40 comporte alors des orifices 41 de tailles différentes, garantissant ainsi une répartition équitable du gaz entre les orifices 41.

Typiquement, pour les débits de gaz secondaire usuels, compris entre 0.1 et 10 l/min, à une température de 10 à 40°C, la résistance à l'écoulement de l'injecteur 40 est telle qu'elle entraine une chute de pression dans l'injecteur 40 inférieure à 5 bar, idéalement inférieure à 1.5 bar.

Le module d'injection 12 de l'invention permet d'assurer un mélange homogène des gaz, sans imposer trop de résistance à l'écoulement du gaz principal, et dans un volume restreint.

Ainsi, après mesure expérimentale, le mélange gazeux obtenu est homogène dans un plan situé à moins de 5 cm du plan d'injection, c'est-à-dire de l'injecteur 40, voire même à moins de 2 cm dans certaines configurations.

A titre comparatif, la figure 8b représente un schéma d'une injection de NO obtenu par simulation numérique dans laquelle un débit secondaire de NO est injecté, comme dans le cas de la Figure 8a, dans un débit principal d'air non turbulent à pression atmosphérique et 25°C, au travers d'un injecteur 40 selon l'invention.

Comme visible, le NO délivré est mélangé de manière homogène au flux principal d'air dans un plan P50 situé à 50 mm en aval de l'injecteur 40.

A noter cependant que, dans les conditions de simulation fixées ici, le mélange n'est pas tout à fait homogène dans un plan P20 situé à 20 mm en aval de l'injecteur 40.

Par ailleurs, comme déjà évoqué, un autre aspect de l'invention concerne le prélèvement de gaz en aval du plan d'injection, c'est-à-dire de l'injecteur 40, au sein du module d'injection 12. Un tel prélèvement est parfois nécessaire afin de mesurer la concentration réelle du mélange gazeux obtenu de manière à en assurer un meilleur contrôle ou à surveiller des concentrations dangereuses pour le patient (formation de NO₂ notamment).

Comme déjà dit, l'injecteur 40 permet d'homogénéiser le mélange à une faible distance de l'injecteur 40, typiquement à une distance comprise entre 2 à 5 cm En effet, plus proche du plan d'injection, le gaz n'est pas encore homogène ou pas totalement homogène. Dès lors, un prélèvement en un point unique situé dans un tel plan ne permettrait pas de réaliser un prélèvement représentatif de gaz, en termes de concentration notamment.

Toutefois, il est souhaitable de limiter au maximum les dimensions du module 12, et un encombrement inférieur à 5 cm est préférable.

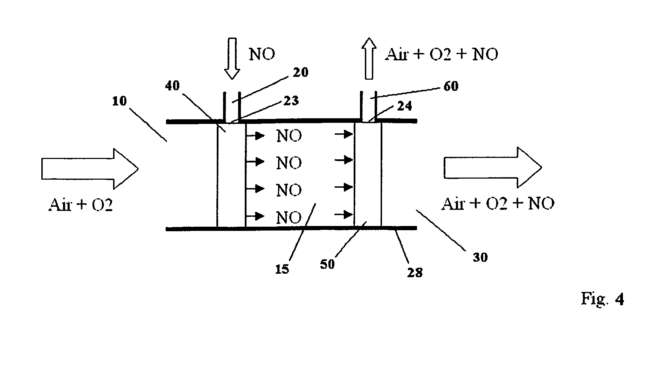

Pour ce faire, comme schématisé en Figure 4, on positionne un dispositif ou une pièce de prélèvement 50 dans la ligne 15, en aval de l'injecteur 40, lequel dispositif de prélèvement 50 est relié fluidiquement à une ligne de prélèvement de gaz 60 débouchant au niveau d'un orifice 24 aménagé dans la paroi 28 de la ligne d'acheminement 15 du module d'injection 12 de l'invention.

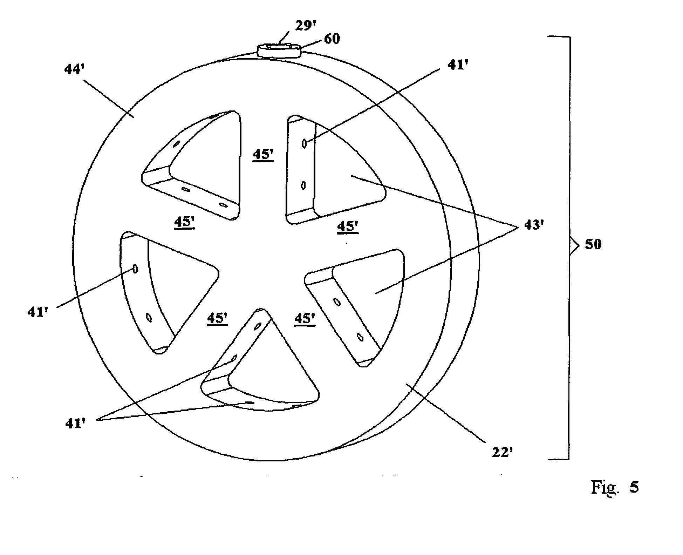

Comme illustré en Figure 5, le dispositif de prélèvement 50 est creux et comprend plusieurs orifices de prélèvement 41' et un orifice de sortie de prélèvement 29' situé en regard de l'orifice 24 du module 12 de manière à ce que le gaz prélevé passe successivement au travers des orifices de prélèvement 41', circule au sein du dispositif de prélèvement 50, puis en ressort via l'orifice de sortie de prélèvement 29' avant d'être évacué vers la ligne de prélèvement 60, via l'orifice 24 pratiqué dans la paroi du corps du module 12, c'est-à-dire dans la paroi de la ligne 15.

La pièce de prélèvement 50 étant munie de plusieurs orifices de prélèvement 41', le gaz prélevé est représentatif, en termes de concentration notamment, du mélange gazeux qui parcourt le conduit 15 dans la section de prélèvement de la pièce 50. Il est alors possible de positionner la pièce de prélèvement 50 à une distance inférieure à 2 cm de l'injecteur 40, en aval de ce dernier.

La pièce de prélèvement 50 peut être ou ne pas être identique à l'injecteur 40, tant par la géométrie que par le nombre, la taille et la disposition des orifices. Selon un mode de réalisation préféré, le dispositif de prélèvement 50 et l'injecteur 40 sont identiques ou quasi-identiques. Ainsi, ils pourront tous deux avoir une forme générale de roue, comme expliqué ci-avant et illustré en Figures 3a, 3b et 5.

Typiquement, le débit de prélèvement est inférieur à 1 l/min, par exemple 100 ml/min, ce qui permet, selon un autre mode de réalisation, de diminuer la taille des orifices de prélèvement et augmenter la surface évidée 43' de la pièce de prélèvement 50, réduisant ainsi la résistance à l'écoulement du gaz au travers de la pièce de prélèvement 50.

Les orifices de prélèvement 41' du dispositif 50 de prélèvement peuvent être ou ne pas être positionnés en regard des orifices d'injection 41 de l'injecteur 40, c'est-à-dire décalés ou non angulairement les uns par rapport aux autres et par rapport à l'axe du conduit 15 du module d'injection 12 de l'invention.

Couplée à l'injecteur 40, le dispositif de prélèvement multi-orifices 50 permet de réaliser un prélèvement représentatif, en termes de concentration du mélange gazeux, dans un plan aval du plan d'injection et à proximité de ce dernier, y compris à moins de 5 cm, voire à moins de 2 cm de celui-ci, comme illustrée en figure 4.

Selon encore un autre mode de réalisation de l'invention, le module d'injection 12 de l'invention permet également d'opérer une mesure du débit de gaz circulant au travers du module d'injection 12, ce qui permet alors d'assurer par exemple un contrôle précis du débit de gaz secondaire délivré.

Ceci est particulièrement avantageux dans le cas d'une administration de NO qui doit être dosé avec précision, notamment lorsqu'on l'utilise pour le traitement des nouveau-nés souffrant d'hypertension pulmonaire.

Comme déjà expliqué, la résistance à l'écoulement du gaz par l'introduction de l'injecteur 40 et/ou de la pièce de prélèvement 50 est faible mais non nulle.

Dès lors, on peut mettre à profit cette résistance qui se traduit par une perte de charge entre l'amont et l'aval de l'injecteur 40 et/ou de la pièce de prélèvement 50, pour effectuer une mesure de débit du gaz circulant dans le conduit 15 du module 12.

Plus précisément, le débit du gaz peut être déterminé par mesure de la différence de pression entre l'amont et l'aval de l'injecteur 40 et/ou de la pièce de prélèvement 50.

Cette différence de pression est d'autant plus grande que le débit de gaz principal est élevé.

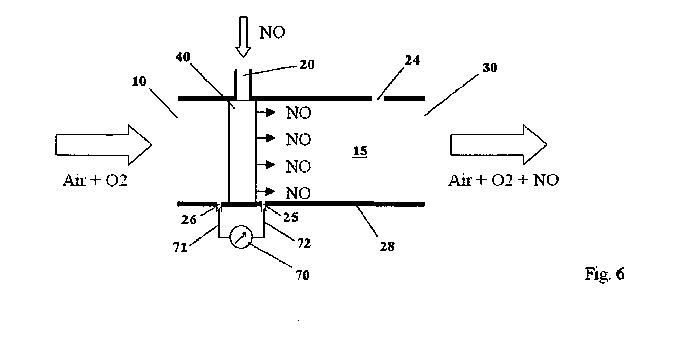

Pour ce faire, dans ce mode de réalisation, le module d'injection 12 comporte deux orifices supplémentaires 25, 26 pratiqués dans la paroi 28 périphérique du corps de module et coopérant avec des lignes de mesure de pression 71, 72, et agencés de part et d'autre de l'injecteur 40 (et/ou de la pièce de prélèvement 50), permettant la mesure des pressions, par exemple au travers d'un capteur de pression différentielle 70, comme illustré en Figure 6.

Un tel capteur 70 est alors relié électriquement à une carte de commande du système de pilotage du dispositif de délivrance 1 de gaz, par exemple de NO, laquelle carte de commande traite les signaux de pression délivrés par les lignes de mesure de pression 71, 72 et en déduit le débit instantané de gaz. Celui-ci peut alors être utilisé pour ajuster la quantité de NO/N₂ délivré par l'injecteur 40 de manière à obtenir la teneur finale en NO désirée dans le mélange qui se forme dans le module d'injection 12.

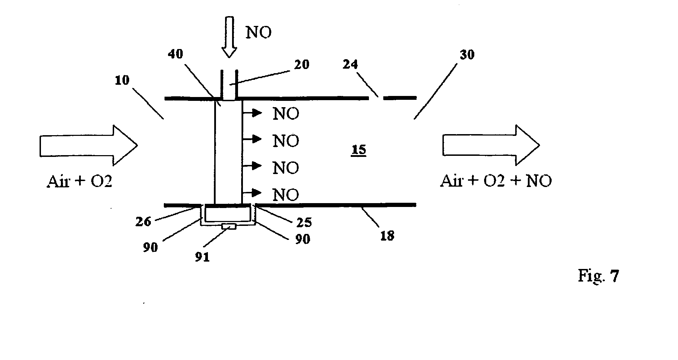

De manière alternative, il est à noter que, selon encore un autre mode de réalisation schématisé en Figure 7, une mesure du débit peut également être réalisée dans un conduit de dérivation 90, c'est-à-dire une ligne de bipasse 90, venant se raccorder au conduit 15, par exemple au niveau des orifices 25, 26, et dans lequel une fraction connue et fixe du débit principal s'écoule.

Un organe de mesure de débit 91, par exemple de technologie massique, permet alors de déterminer le débit dans le conduit de dérivation 90 et donc de connaître le débit de gaz principal.

La résistance à l'écoulement de l'injecteur 40 et/ou de la pièce 50 de prélèvement est/sont, dans cette configuration, mises à profit pour maîtriser la part de débit qui s'écoule dans le conduit de dérivation 90.

Le conduit de dérivation 90 supplémentaire et l'organe de mesure de débit 91 situé sur ce conduit 90 sont reliés électriquement à la carte de commande du système de pilotage du dispositif 1 de délivrance de gaz, tel du NO, comme expliqué ci-dessus, afin d'y traiter le signal mesuré et l'utiliser pour piloter l'apport en gaz, en particulier en NO.

Le module d'injection 12 de l'invention va s'insérer dans le circuit patient, c'est-à-dire sur la canalisation principale 5 reliant, via son extrémité amont 5a, le ventilateur 4 au patient, via une interface patient 8, tel un masque ou analogue, agencée à l'extrémité aval 5b de la canalisation principale 5.

Un humidificateur 3 de gaz est par ailleurs, si une humidification des gaz est souhaitée pour le patient, agencé entre la première source de gaz 4 et le module d'injection 12. Cet humidificateur est donc optionnel.

De préférence, un raccord à 3-voies 7, telle une pièce en Y ou en T, est agencé à l'extrémité aval 5b de la canalisation principale 5, et permet de raccorder l'extrémité aval 5b de la canalisation 15 à l'interface patient 8.

Le module d'injection 12 est agencé de manière fluidique entre l'humidificateur 3 et le raccord à 3-voies 7 de sorte que le gaz traverse successivement le module 12, l'humidificateur 3, le raccord 3-voies 7 avant d'atteindre l'interface patient 8.

D'une façon générale, le module d'injection 12 de l'invention peut être utilisé par tout type de gaz et son utilisation n'est donc nullement limitée aux mélanges gazeux à base de NO, bien que le module d'injection de l'invention soit particulièrement bien adapté à un tel usage.

## Revendications

1. Module d'injection de gaz (12) comprenant :
- un corps de module traversé par une ligne d'acheminement de gaz (15) entre une extrémité d'entrée de gaz (10) et une extrémité de sortie de gaz (30), ladite ligne d'acheminement de gaz (15) étant délimitée par une paroi périphérique (28), et
- un injecteur de gaz (40) formé d'un élément creux (22) comprenant un orifice d'entrée (29) de gaz permettant à un gaz de pénétrer dans l'élément creux (22) et plusieurs orifices de sortie (41) de gaz permettant au gaz de sortir de l'élément creux (22),
et dans lequel :
- le corps de module comporte un premier orifice latéral (23) aménagé dans la paroi périphérique (28) de la ligne d'acheminement de gaz (15) et au moins un deuxième orifice latéral (24, 25, 26) aménagé dans la paroi périphérique (28) de la ligne d'acheminement de gaz (15),
- ledit injecteur de gaz (40) est agencé dans la ligne d'acheminement de gaz (15) de sorte que le premier orifice latéral (23) soit situé en vis-à-vis de l'orifice d'entrée (29) de gaz de l'élément creux (22) et qu'au moins une partie des orifices de sortie (41) de gaz soient agencés de manière à distribuer du gaz en plusieurs points d'injection de la section de la ligne d'acheminement de gaz (15), et
- ledit au moins un deuxième orifice latéral (24, 25, 26) étant aménagé dans la paroi périphérique (28) de la ligne d'acheminement de gaz (15), entre l'injecteur de gaz (40) et l'extrémité de sortie de gaz (30) de ladite ligne d'acheminement de gaz (15).

2. Module selon la revendication précédente, **caractérisé en ce que** l'injecteur de gaz (40) comporte entre 5 et 100 orifices de sortie (41) de gaz.

3. Module selon l'une des revendications précédentes, **caractérisé en ce que** ledit injecteur de gaz (40) a une forme annulaire ou semi-annulaire et/ou les orifices (41) ont un diamètre compris entre 0.1 et 3 mm.

4. Module selon l'une des revendications précédentes, **caractérisé en ce que** l'injecteur de gaz (40) a une forme de roue comprenant un cadre creux périphérique (44) et *n* rayons creux (45), avec *n* compris entre 3 et 20.

5. Module selon l'une des revendications précédentes, **caractérisé en ce que** la somme des surfaces évidées (43) situées dans le cadre creux (44) représente au moins 50% de la section de passage de la ligne d'acheminement (15).

6. Module selon l'une des revendications précédentes, **caractérisé en ce que** le corps de module (21) comporte un deuxième orifice latéral (24) et au moins un troisième orifice latéral (25, 26) aménagés dans la paroi périphérique (28) de la ligne d'acheminement de gaz (15), au moins un (26) desdits orifices latéraux étant agencé entre l'injecteur de gaz (40) et l'extrémité d'entrée de gaz (10) de la ligne d'acheminement de gaz (15), et au moins un desdits orifices latéraux (24, 25) étant agencé entre l'injecteur de gaz (40) et l'extrémité de sortie de gaz (30) de la ligne d'acheminement de gaz (15).

7. Module selon l'une des revendications précédentes, **caractérisé en ce que** le corps de module (21) comporte au moins un deuxième orifice latéral (24), un troisième orifice latéral (25) et un quatrième orifice latéral (26) aménagés dans la paroi périphérique (28) de la ligne d'acheminement de gaz (15), le deuxième orifice latéral (24) et le troisième orifice latéral (25) étant agencés entre l'injecteur de gaz (40) et l'extrémité de sortie de gaz (30) de la ligne d'acheminement de gaz (15), et le quatrième orifice latéral (26) étant agencé entre l'injecteur de gaz (40) et l'extrémité d'entrée de gaz (10) de la ligne d'acheminement de gaz (15).

8. Module selon l'une des revendications précédentes, **caractérisé en ce qu'**un dispositif de prélèvement de gaz (50) est agencé dans la ligne d'acheminement de gaz (15) entre l'injecteur de gaz (40) et l'extrémité de sortie de gaz (30) de la ligne d'acheminement de gaz (15), ledit dispositif de prélèvement de gaz (50) coopérant avec le deuxième orifice latéral (24).

9. Module selon l'une des revendications précédentes, **caractérisé en ce que** le dispositif de prélèvement de gaz (50) est formé d'un second élément creux (22') comprenant plusieurs orifices d'entrée (41') de gaz permettant à du gaz circulant dans la ligne d'acheminement de gaz (15) de pénétrer dans l'élément creux (22') et un orifice de sortie (29') de gaz permettant au gaz de sortir de l'élément creux (22') via le deuxième orifice latéral (24).

10. Module selon l'une des revendications précédentes, **caractérisé en ce que** l'injecteur de gaz (40) et le dispositif de prélèvement de gaz (50) sont identiques.

11. Installation de distribution d'un mélange gazeux comprenant un premier gaz et un deuxième gaz, comportant :
- une première source de gaz (4) pour distribuer un premier gaz dans une ligne principale de distribution de gaz (5) reliée fluidiquement à ladite première source de gaz (4),
- un module d'injection de gaz (12) selon l'une des revendications précédentes agencé sur la ligne principale de distribution de gaz (5) de manière à ce que la ligne principale de distribution de gaz (5) et la ligne d'acheminement de gaz (15) du module soient en communication fluidique, et
- une deuxième source de gaz (1) en communication fluidique, via le premier orifice latéral (23), avec l'orifice d'entrée (29) de gaz de l'injecteur de gaz (40) de manière à alimenter l'injecteur de gaz (40) en un deuxième gaz issu de la deuxième source de gaz (1).

12. Installation selon la revendication 11, **caractérisée en ce que** la première source de gaz (4) comprend un ventilateur respiratoire délivrant de l'air enrichi ou non en oxygène, ou un mélange N₂/O₂.

13. Installation selon l'une des revendications 11 ou 12, **caractérisée en ce que** la deuxième source de gaz (1) comporte est une source de NO ou d'un mélange gazeux contenant du NO.

14. Installation selon l'une des revendications 11 à 13, **caractérisée en ce qu'**elle comporte en outre un humidificateur (3) de gaz agencé entre la première source de gaz (4) et le module d'injection (12), de préférence elle comporte en outre un raccord à 3-voies (7), telle une pièce en Y ou en T, agencé à l'extrémité aval (5b) de la canalisation principale (5), le module d'injection (12) étant agencé entre l'humidificateur (3) et le raccord à 3-voies (7).

15. Installation selon l'une des revendications 11 à 14, **caractérisée en ce qu'**elle comporte en outre une ligne de prélèvement de gaz (60) connectée au deuxième orifice latéral (24), et/ou des lignes de mesure de pression (71, 72) connectées au troisième et au quatrième orifice latéral (25, 26).

## Patentansprüche

1. Gasinjektionsmodul (12), das Folgendes umfasst:
- einen Modulkörper, durch den eine Gastransportleitung (15) zwischen einem Gaseingangsende (10) und einem Gasausgangsende (30) verläuft, wobei die Gastransportleitung (15) durch eine periphere Wand (28) begrenzt wird, und
- einen Gasinjektor (40), der von einem hohlen Element (22) gebildet wird, das einen Gaseinlass (29), durch den ein Gas in das hohle Element (22) eindringen kann, sowie mehrere Gasauslässe (41) umfasst, so dass Gas aus dem hohlen Element (22) austreten kann,
und wobei:
- der Modulkörper eine in der peripheren Wand (28) der Gastransportleitung (15) vorgesehene erste seitliche Öffnung (23) und wenigstens eine in der peripheren Wand (28) der Gastransportleitung (15) vorgesehene zweite seitliche Öffnung (24, 25, 26) aufweist,
- der Gasinjektor (40) in der Gastransportleitung (15) so vorgesehen ist, dass sich die erste seitliche Öffnung (23) gegenüber dem Gaseinlass (29) des hohlen Elements (22) befindet, und so, dass wenigstens ein Teil der Gasauslässe (41) so vorgesehen sind, dass Gas an mehreren Injektionsstellen des Abschnitts der Gastransportleitung (15) verteilt wird, und
- wenigstens eine zweite seitliche Öffnung (24, 25, 26) in der peripheren Wand (28) der Gastransportleitung (15) zwischen dem Gasinjektor (40) und dem Gasaustrittsende (30) der Gastransportleitung (15) vorgesehen ist.

2. Modul nach dem vorherigen Anspruch, **dadurch gekennzeichnet, dass** der Gasinjektor (40) zwischen 5 und 100 Gasauslässe (41) umfasst.

3. Modul nach einem der vorherigen Ansprüche, **dadurch gekennzeichnet, dass** der Gasinjektor (40) eine ringförmige oder halbringförmige Gestalt hat und/oder die Öffnungen (41) einen Durchmesser zwischen 0,1 und 3 mm haben.

4. Modul nach einem der vorherigen Ansprüche, **dadurch gekennzeichnet, dass** der Gasinjektor (40) eine Räderform mit einem peripheren Hohlrahmen (44) und *n* hohlen Speichen (45) hat, wobei *n* zwischen 3 und 20 ist.

5. Modul nach einem der vorherigen Ansprüche, **dadurch gekennzeichnet, dass** die Summe der ausgesparten Flächen (43) in dem Hohlrahmen (44) wenigstens 50 % des Passagequerschnitts der Transportleitung (15) beträgt.

6. Modul nach einem der vorherigen Ansprüche, **dadurch gekennzeichnet, dass** der Modulkörper (21) eine zweite seitliche Öffnung (24) und wenigstens eine dritte seitliche Öffnung (25, 26) umfasst, die in der peripheren Wand (28) der Gastransportleitung (15) vorgesehen ist, wobei wenigstens eine (26) der seitlichen Öffnungen zwischen dem Gasinjektor (40) und dem Gaseintrittsende (10) der Gastransportleitung (15) vorgesehen ist und wenigstens eine der seitlichen Öffnungen (24, 25) zwischen dem Gasinjektor (40) und dem Gasaustrittsende (30) der Gastransportleitung (15) vorgesehen ist.

7. Modul nach einem der vorherigen Ansprüche, **dadurch gekennzeichnet, dass** der Modulkörper (21) wenigstens eine zweite seitliche Öffnung (24), eine dritte seitliche Öffnung (25) und eine vierte seitliche Öffnung (26) umfasst, die in der peripheren Wand (28) der Gastransportleitung (15) vorgesehen sind, wobei die zweite seitliche Öffnung (24) und die dritte seitliche Öffnung (25) zwischen dem Gasinjektor (40) und dem Gasaustrittsende (30) der Gastransportleitung (15) vorgesehen sind und wobei die vierte seitliche Öffnung (26) zwischen dem Gasinjektor (40) und dem Gaseintrittsende (10) der Gastransportleitung (15) vorgesehen ist.

8. Modul nach einem der vorherigen Ansprüche, **dadurch gekennzeichnet, dass** eine Gasentnahmevorrichtung (50) in der Gastransportleitung (15) zwischen dem Gasinjektor (40) und dem Gasaustrittsende (30) der Gastransportleitung (15) vorgesehen ist, wobei die Gasentnahmevorrichtung (50) mit der zweiten seitlichen Öffnung (24) zusammenwirkt.

9. Modul nach einem der vorherigen Ansprüche, **dadurch gekennzeichnet, dass** die Gasentnahmevorrichtung (50) von einem zweiten hohlen Element (22') gebildet wird, das mehrere Gaseinlässe (41') umfasst, die es zulassen, dass in der Gastransportleitung (15) zirkulierendes Gas in das hohle Element (22') eindringt, und einen Gasauslass (29'), der den Austritt von Gas aus dem hohlen Element (22') über die zweite seitliche Öffnung (24) zulässt.

10. Modul nach einem der vorherigen Ansprüche, **dadurch gekennzeichnet, dass** der Gasinjektor (40) und die Gasentnahmevorrichtung (50) identisch sind.

11. Anlage zum Verteilen eines gasförmigen Gemischs, das ein erstes Gas und ein zweites Gas umfasst, die Folgendes umfasst:
- eine erste Gasquelle (4) zum Verteilen eines ersten Gases in einer Gasverteilungshauptleitung (5), die fluidisch mit der ersten Gasquelle (4) verbunden ist,
- ein Gasinjektionsmodul (12) nach einem der vorherigen Ansprüche, das auf der Gasverteilungshauptleitung (5) so angeordnet ist, dass die Gasverteilungshauptleitung (5) und die Gastransportleitung (15) des Moduls in Fluidverbindung miteinander sind, und
- eine zweite Gasquelle (1) in Fluidverbindung, über die erste seitliche Öffnung (23), mit dem Gaseinlass (29) des Gasinjektors (40), um den Gasinjektor (40) mit einem zweiten Gas aus der zweiten Gasquelle (1) zu versorgen.

12. Anlage nach Anspruch 11, **dadurch gekennzeichnet, dass** die erste Gasquelle (4) einen Ansauglüfter umfasst, der mit Sauerstoff angereicherte oder nicht angereicherte Luft oder ein N₂/O₂-Gemisch zuführt.

13. Anlage nach Anspruch 11 oder 12, **dadurch gekennzeichnet, dass** die zweite Gasquelle (1) eine Quelle von NO oder einem NO enthaltenden gasförmigen Gemisch umfasst.

14. Anlage nach einem der Ansprüche 11 bis 13, **dadurch gekennzeichnet, dass** sie ferner einen Gasbefeuchter (3) umfasst, der zwischen der ersten Gasquelle (4) und dem Injektionsmodul (12) angeordnet ist und vorzugsweise darüber hinaus einen 3-Wege-Anschluss (7) umfasst, wie z.B. ein Y- oder T-Stück, das am stromabwärtigen Ende (5b) des Hauptkanals (5) angeordnet ist, wobei das Injektionsmodul (12) zwischen dem Befeuchter (3) und dem 3-Wege-Anschluss (7) vorgesehen ist.

15. Anlage nach einem der Ansprüche 11 bis 14, **dadurch gekennzeichnet, dass** sie ferner eine Gasentnahmeleitung (60) umfasst, die mit der zweiten seitlichen Öffnung (24) verbunden ist, und/oder Druckmessleitungen (71, 72), die mit der dritten und der vierten seitlichen Öffnung (25, 26) verbunden sind.

## Claims

1. Gas injection module (12) comprising:
- a module body through which a gas transport line (15) passes between a gas inlet end (10) and a gas outlet end (30), said gas transport line (15) being delimited by a peripheral wall (28), and
- a gas injector (40) formed by a hollow element (22), comprising a gas inlet opening (29) allowing a gas to penetrate the hollow element (22) and a plurality of gas outlet openings (41) allowing the gas to exit the hollow element (22), and wherein:
- the module body comprises a first lateral opening (23) made in the peripheral wall (28) of the gas transport line (15) and at least a second lateral opening (24, 25, 26) made in the peripheral wall (28) of the gas transport line (15),
- said gas injector (40) is arranged in the gas transport line (15) so that the first lateral opening (23) is positioned opposite the gas inlet opening (29) of the hollow element (22) and so that at least some of the gas outlet openings (41) are arranged so as to distribute gas to a plurality of injection points of the gas transport line (15), and
- said at least one second lateral opening (24, 25, 26) being made in the peripheral wall (28) of the gas transport line (15), between the gas injector (40) and the gas outlet end (30) of said gas transport line (15).

2. Module according to the preceding claim, **characterised in that** the gas injector (40) comprises between 5 and 100 gas outlet openings (41).

3. Module according to any of the preceding claims, **characterised in that** said gas injector (40) is annular or semi-annular and/or the openings (41) have a diameter of between 0.1 and 3 mm.

4. Module according to any of the preceding claims, **characterised in that** the gas injector (40) is in the shape of a wheel comprising a peripheral hollow frame (44) and *n* hollow spokes (45), where *n* is between 3 and 20.

5. Module according to any of the preceding claims, **characterised in that** the total of the empty surface area (43) of the hollow frame (44) represents at least 50 % of the flow area of the transport line (15).

6. Module according to any of the preceding claims, **characterised in that** the module body (21) comprises a second lateral opening (24) and at least a third lateral opening (25, 26) made in the peripheral wall (28) of the gas transport line (15), at least one (26) of said lateral openings being arranged between the gas injector (40) and the gas inlet end (10) of the gas transport line (15), and at least one of said lateral openings (24, 25) being arranged between the gas injector (40) and the gas outlet end (30) of the gas transport line (15).

7. Module according to any of the preceding claims, **characterised in that** the module body (21) comprises at least a second lateral opening (24), a third lateral opening (25) and a fourth lateral opening (26) made in the peripheral wall (28) of the gas transport line (15), the second lateral opening (24) and the third lateral opening (25) being arranged between the gas injector (40) and the gas outlet end (30) of the gas transport line (15), and the fourth lateral opening (26) being arranged between the gas injector (40) and the gas inlet end (10) of the gas transport line (15).

8. Module according to any of the preceding claims, **characterised in that** a gas withdrawal device (50) is arranged in the gas transport line (15) between the gas injector (40) and the gas outlet end (30) of the gas transport line (15), said gas withdrawal device (50) cooperating with the second lateral opening (24).

9. Module according to any of the preceding claims, **characterised in that** the gas withdrawal device (50) is formed by a second hollow element (22') comprising a plurality of gas inlet openings (41') allowing gas circulating in the gas transport line (15) to penetrate the hollow element (22'), and a gas outlet opening (29') allowing the gas to exit the hollow element (22') via the second lateral opening (24).

10. Module according to any of the preceding claims, **characterised in that** the gas injector (40) and the gas withdrawal device (50) are identical.

11. Unit for distributing a gas mixture comprising a first gas and a second gas, comprising:
- a first source of gas (4) for distributing a first gas in a main gas distribution line (5) that is in fluid communication with said first source of gas (4),
- a gas injection module (12) according to any of the preceding claims, arranged on the main gas distribution line (5) so that the main gas distribution line (5) and the gas transport line (15) of the module are in fluid communication, and
- a second source of gas (1) in fluid communication with the gas inlet opening (29) of the gas injector (40) via the first lateral opening (23) so as to supply the gas injector (40) with a second gas from the second source of gas (1).

12. Unit according to claim 11, **characterised in that** the first source of gas (4) comprises a respiratory ventilator which delivers oxygen-enriched or non-oxygen-enriched air or an N₂/O₂ mixture.

13. Unit according to either claim 11 or claim 12, **characterised in that** the second source of gas (1) comprises is a source of NO or a gas mixture containing NO.

14. Unit according to any of claims 11 to 13, **characterised in that** it further comprises a gas humidifier (3) arranged between the first source of gas (4) and the injection module (12), and it preferably further comprises a three-way connector (7), such as a Y-shaped or T-shaped part, arranged at the downstream end (5b) of the main pipeline (5), the injection module (12) being arranged between the humidifier (3) and the three-way connector (7).

15. Unit according to any of claims 11 to 14, **characterised in that** it further comprises a gas withdrawal line (60) connected to the second lateral opening (24), and/or pressure measurement lines (71, 72) connected to the third and to the fourth lateral opening (25, 26).
